# EUROPÄISCHE PATENTSCHRIFT

(11) **EP 3 244 937 B1**
(45) Veröffentlichungstag und Bekanntmachung des Hinweises auf die Patenterteilung: **15.05.2024**
(21) Anmeldenummer: 16708072.0
(22) Anmeldetag: 11.01.2016
(51) Int. Cl.: A61L 27/06, A61C 8/02, A61L 31/02, A61L 31/06, A61F 2/28, A61F 2/30, G16H 50/50, G16H 70/20

(54) **VERFAHREN ZUR HERSTELLUNG EINES AUFSATZES EINER ABDECKVORRICHTUNG FÜR EINE KNOCHENDEFEKTSTELLE; VORRICHTUNG ZUR ABDECKUNG UND/ODER REKONSTRUKTION EINER KNOCHENDEFEKTSTELLE**
METHOD FOR PRODUCING AN ATTACHMENT PIECE OF A COVER DEVICE FOR A BONE DEFECT SITE, AND DEVICE FOR COVERING AND/OR RECONSTRUCTING A BONE DEFECT SITE
PROCÉDÉ POUR LA FABRICATION D'UN CHAPEAU D'UN DISPOSITIF DE RECOUVREMENT POUR UN SITE DE DÉFAUT OSSEUX, ET DISPOSITIF POUR LE RECOUVREMENT ET/OU LA RECONSTRUCTION D'UN SITE DE DÉFAUT OSSEUX

(30) Priorität: 13.01.2015 DE 102015000496; 08.05.2015 DE 102015006153
(43) Veröffentlichungstag der Anmeldung: 22.11.2017
(73) Patentinhaber: Reoss GmbH, 70794 Filderstadt (DE)
(72) Erfinder: SEILER, Marcus, 70597 Stuttgart (DE)
(74) Vertreter: Patentanwälte Schuster, Müller & Partner mbB
(86) Internationale Anmeldenummer: PCT/DE2016/000016
(87) Internationale Veröffentlichungsnummer: WO 2016/112894

(56) Entgegenhaltungen:
- WO-A1-00/59409
- WO-A1-2011/100951
- WO-A2-2010/023665
- DE-A1- 4 102 462
- DE-U1-202015 005 610
- US-B1- 7 172 422
- Marcus Seiler: "CAD/CAM-Lösung für die patientenspezifische 3D-Knochenregeneration", DI international Dentale Implantologie und Parodontologie | DImagazin-aktuell.de, 7. Oktober 2015 (2015-10-07), XP055266968, Gefunden im Internet: URL:http://www.dimagazin-aktuell.de/implan tologie/knochenmanagement/story/cadcam-loe sung-fuer-die-patientenspezifische-3d-knoc henregeneration__3265.html [gefunden am 2016-04-20]

## Beschreibung

### Stand der Technik

Die Erfindung geht aus von einem Verfahren zur Herstellung eines Aufsatzes einer Abdeckvorrichtung für eine Knochendefektstelle, nach der Gattung des Anspruchs 1, und einer Vorrichtung zur Abdeckung und/oder Rekonstruktion einer Knochendefektstelle, nach der Gattung des Anspruchs 13.

Knochendefektstellen in Form von Ausnehmungen oder Höhlungen im körpereigenen Knochengewebe werden in der Knochenchirurgie, beispielsweise bei der Rekonstruktion von Knochen in der orthopädischen, neurochirurgischen oder plastischen Chirurgie oder bei kieferchirurgischen Operationen, oftmals mit Knochenaufbaumaterial gefüllt. In der Regel besteht das Knochenaufbaumaterial aus einer Mischung aus synthetischen Knochenersatzmaterial (z.B. Hydroxylapatitgranulat) und körpereigenen Knochenpartikeln. Damit das Knochenaufbaumaterial im Wesentlichen ausschließlich von der Knochenseite her knöchern durchwachsen wird, wird die Ausnehmung, wie in der Patentschrift DE 43 02 708 C2 beschrieben, mit einer Abdeckmembran verschlossen. Befestigt wird die Abdeckmembran mit Befestigungsnägeln am körpereigenen Knochen, wobei, da die Abdeckmembran aus einem flexiblen Material besteht, die Befestigung ein Höchstmaß an handwerklichem Geschick des Chirurgen erfordert.

Um diesen Nachteil einer fehlenden Stützfunktion der Abdeckmembran zu überwinden, wird in der Patentschrift US 48 16 339 eine Abdeckmembran beschrieben, die aus mehreren Schichten besteht, wobei diese Schichten nicht aus resorbierbarem Membranmaterial bestehen. Dabei ist es gegebenenfalls erforderlich, dass nach dem Ausheilen des Knochendefektes ein zweiter Eingriff notwendig ist, um körperfremdes Material zu entfernen.

In der Patentschrift DE 10 2005 039 382 B4 wird ein biodegradierbarer Hohlkörper, der insbesondere eine hohlzylindrische oder kegelzylindrische Form aufweist, vorgeschlagen. Der Hohlkörper weist in seinen Wandungen eine Mehrzahl von Öffnungen auf, durch die eine Aufnahme von Blut und damit der Aufbau von Eigenknochen möglich ist. Nachteilig ist hierbei, dass zum Einsetzen des Hohlkörpers eine zylindrische Bohrung mittels eines Bohrers in den vorhandenen Knochen eingebracht werden muss.

In der Offenlegungsschrift DE 10 2006 047 054 A1 wird ein Implantatlager vorgeschlagen, dass sich durch eine hohe Passgenauigkeit und Stabilität auszeichnet, so dass der behandelnde Arzt es einfach handhaben und implantieren kann. Das aus Hydroxylapatit angefertigte Implantatlager, das zum Schutz der Schleimhaut vor mechanischen Einwirkungen und zum Schutz des Implantatlagers vor einwachsenden Gewebe von Seiten der Schleimhaut auf der der Schleimhaut zugewandten Seite eine dünne, insbesondere aus resorbierbaren Material bestehende, Membran aufweist, wird mit einem aufbauenden Fertigungsverfahren hergestellt, so dass die Materialbeschaffenheit eine "Gradientenstruktur" im Sinne einer insbesondere nach innen abnehmenden Dichte bildet. Dabei ist an der dem Knochen zugewandten Seite eine Bauweise mit einer insbesondere porösen Struktur und an der Außenseite des Implantatlagers, an der sich eine Struktur zur Halterung eines Zahnimplantats und/oder eines Zahnersatzes befindet, eine kompakte Bauweise vorgesehen.

Des Weiteren werden in den Offenlegungsschriften DE 198 30 992 A1, DE 10 2005 060 761 A1, DE 41 02 462 A1, DE 42 26 465 A1, WO 00/59409 A1, WO 01/91818 A1, DE 10 2005 041 412 A1, DE 10 2006 047 054 A1, US 2011/0151400 A1, und WO 2006/051401 A2 und WO 2010/023665 A2 und der Patentschrift US 7 172 422 B1 Vorrichtungen für eine Knochendefektstelle beschrieben, wobei sämtliche dieser Lösungen den Nachteil aufweisen, dass sie den neben der Knochendefektstelle vorhandenen gesunden Knochen in Mitleidenschaft ziehen.

In den Offenlegungsschriften DE 10 2011 011 191 A1 und WO 2011/100951 A1 werden Verfahren zur Herstellung eines Aufsatzes einer Abdeckvorrichtung für eine Knochendefektstelle beschrieben, bei denen in einem ersten Verfahrensschritt ein Datensatz aufgenommen wird, der die betroffene Knochendefektstelle in seiner Dreidimensionalität repräsentiert. Anschließend wird der Datensatz zur Planung des Aufsatzes verwendet. Nach einer Umsetzung der Planung des Aufsatzes in einen Planungsdatensatz wird der Planungsdatensatz an ein computergesteuertes Fertigungsverfahren zugeführt, so dass dadurch der Aufsatz aus einem formstabilen Material gebildet wird, wobei dessen dem Knochendefekt zugewandte Wandung oder dessen dem Knochendefekt abgewandte Wandung der Form des regenerierten Knochens entspricht. Nachteilig ist hierbei, dass die Form des regenerierten Knochens auf Grundlage der beim ersten Verfahrensschritt vorhandenen Knochendefektstelle berechnet wird, so dass die Form des regenerierten Knochens auf einer spekulativen Annahme besteht, wie der regenerierte Knochen aussehen sollte. Dabei kann aber ggfls. nicht ausgeschlossen werden, dass die berechnete Form des regenerierten Knochens von der gewünschten Idealform des regenerierten Knochens (Soll-Zustand bzw. Urzustand) abweicht.

Die Offenlegungsschrift WO 00/59409 A1 beschreibt eine Vorrichtung zur Knochengeweberegenerierung mittels Knochenersatzmaterial sowie ein Verfahren zu dessen Herstellung. Die Vorrichtung umfasst zwei z.B. U-förmige Flansche, die lösbar an einem der Knochendefektstelle benachbarten Knochenbereich befestigt werden und mehrere mit diesen verbundene Stabelemente. In den von den Flanschen und den Stabelementen gebildeten Innenraum wird Knochenersatzmaterial eingebracht, welches das Wachstum von neuem Knochen ermöglicht. Die Flansche weisen Schwächungsbereiche auf, die beim Entfernen der Flansche aufgebogen oder aufgebrochen werden (Sollbruchstellen).

### Die Erfindung und ihre Vorteile

Das erfindungsgemäße Verfahren zur Herstellung eines Aufsatzes einer Abdeckvorrichtung für eine Knochendefektstelle, mit den kennzeichnenden Merkmalen des Anspruchs 1, und die erfindungsgemäße Vorrichtung zur Abdeckung und/oder Rekonstruktion einer Knochendefektstelle, wobei mit dem Begriff "Knochendefektstelle" eine Stelle eines (kranken, deformierten, verletzten, durch Alterungsprozesse veränderten, durch Degeneration (z.B. nach Zahnextraktion, Tumor usw.) veränderten und/oder im Volumen veränderten) Knochens (z.B. Hüfte, Wirbelsäule, Kopf, Kiefer odgl.) eines Menschen oder eines Tieres bezeichnet wird, die von der Form und/oder dem Volumen eines gesunden Knochens abweicht, mit den kennzeichnenden Merkmalen des Anspruchs 13, haben demgegenüber den Vorteil, durch einen Vergleich eines ersten Datensatzes, der die betroffene Knochendefektstelle im Ist-Zustand repräsentiert, mit einem zweiten Datensatz, der den Soll-Zustand eines an der Knochendefektstelle regenerierten Knochens repräsentiert, wobei der zweite Datensatz berechnet wird oder zu einer Zeit aufgenommen wurde, zu der der Knochen an der jetzt zu regenerierenden Stelle noch ein gesunder Knochen war, es ermöglicht dass der regenerierte Knochen, der durch die Regenerierung der Knochendefektstelle entsteht, eine Form aufweist, die der Form entspricht, die der Knochen an der zu regenerierenden Stelle hatte, als er noch gesund war, so dass der zweite Datensatz des gesunden Knochens somit auch auf einer tatsächlichen Messung beruhen kann und nicht, wie durch den Stand der Technik bekannt, nur auf einer Berechnung der Form des zu regenerierenden Knochens. Erfindungsgemäß kann also eine Konservierung des Sollzustandes stattfinden. Dies bedeutet, dass ein Datensatz von einem gesunden Knochen erstellt wurde, um diesen Datensatz im Bedarfsfall (ggfls. Jahre oder Jahrzehnte später), wenn an diesem dokumentierten gesunden Knochen eine Knochendefektstelle aufgetreten ist, heranzuziehen, um zusammen mit dem Datensatz der die aktuelle Knochendefektstelle erfasst, eine geeignete therapeutische Behandlungsmethode einzuleiten, die z.B. die auf den ersten Datensatz und den zweiten Datensatz basierende Herstellung eines Aufsatzes einer Abdeckvorrichtung für die Knochendefektstelle umfasst. Die Sollbruchstelle birgt zudem den Vorteil, dass, sofern der Aufsatz nach einer erfolgreichen Knochenregeneration entfernt werden soll, diese Entfernung minimal invasiv erfolgen kann, ohne "alles offen legen zu müssen", da der Aufsatz aufgrund der Sollbruchstelle in mindestens zwei Teile zerkleinert werden kann. Die Entfernung des Aufsatzes ist somit sehr leicht möglich. Des Weiteren kann die Sollbruchstelle dazu dienen, dass nicht benötigte Teile des Aufsatzes von dem Rest des Aufsatzes abgetrennt werden. Bevorzugt ist der Aufsatz ausschließlich im Bereich der Knochendefektstelle angeordnet und/oder fixiert, so dass er den an die Knochendefektstelle angrenzenden gesunden Knochen, an dem aufgrund seiner Gesundheit sowieso keine Regeneration des Knochens stattfindet, nicht in Mitleidenschaft zieht. Der Aufsatz ist somit bevorzugt passgenau auf die Knochendefektstelle abgestimmt und endet bevorzugt bündig am gesunden Knochen.

Nach einer vorteilhaften Ausgestaltung des erfindungsgemäßen Verfahrens zur Herstellung eines Aufsatzes einer Abdeckvorrichtung für eine Knochendefektstelle, bei dem bevorzugt die rechnergestützte Formgebung (CAD) des Aufsatzes mit einer rechnergestützten Fertigung (CAM) zu CAD/CAM kombiniert wird, so dass ein am Computer entwickeltes Entwurfsmodell des Aufsatzes direkt elektronisch an die Fertigung übermittelt wird, bestehend aus folgenden Verfahrensschritten:
- Aufnahme eines ersten Datensatzes, der die betroffene Knochendefektstelle im Ist-Zustand repräsentiert,
- Vergleich des ersten Datensatzes mit einem zweiten Datensatz, der den Soll-Zustand eines an der Knochendefektstelle regenerierten Knochens repräsentiert, wobei der zweite Datensatz durch Berechnung entsteht oder zu einer Zeit aufgenommen wurde, zu der der Knochen an der jetzt zu regenerierenden Stelle noch ein gesunder Knochen war, und
- Verwendung des ersten Datensatzes und des zweiten Datensatzes zur Planung des Aufsatzes, der eine dem Knochendefekt abgewandte Wandung (Oberfläche) und eine dem Knochendefekt zugewandte Wandung (Oberfläche) aufweist, und ggfls. mit mindestens einem Fixiermittel an einem Knochen fixierbar ist,

- Umsetzung der Planung des Aufsatzes in einen Planungsdatensatz und
- Zuführung des Planungsdatensatzes an ein Fertigungsverfahren, insbesondere an ein computergesteuertes Fertigungsverfahren, in dem der Aufsatz aus einem formstabilen Material gebildet wird und dessen dem Knochendefekt zugewandte Wandung (Oberfläche) oder dessen dem Knochendefekt abgewandte Wandung (Oberfläche) der Form des regenerierten Knochens im zuvor ermittelten Soll-Zustand entspricht, wobei während der Fertigung des Aufsatzes mindestens eine auftrennbare Sollbruchstelle zur Entfernung des Aufsatzes an dem Aufsatz angeordnet wird und der Aufsatz entlang einer Öffnung, die sich durch Auftrennen der mindestens einen Sollbruchstelle bildet, so dimensioniert ist, dass der Aufsatz bündig an den benachbarten gesunden Knochen einer zuvor ermittelten Knochendefektstelle abschließen kann,
wobei die Aufnahme des ersten Datensatzes die betroffene Knochendefektstelle in ihrer Dreidimensionalität repräsentiert und/oder die Aufnahme des zweiten Datensatzes die Form des noch gesunden Knochens in seiner Dreidimensionalität repräsentiert.

Nach einer zusätzlichen vorteilhaften Ausgestaltung des erfindungsgemäßen Verfahrens zur Herstellung eines Aufsatzes einer Abdeckvorrichtung für eine Knochendefektstelle erfolgen die Aufnahme des ersten Datensatzes, der den Ist-Zustand repräsentiert, und/oder die Aufnahme des zweiten Datensatzes, der den Soll-Zustand repräsentiert, durch mindestens ein bildgebendes Verfahren.

Nach einer zusätzlichen vorteilhaften Ausgestaltung des erfindungsgemäßen Verfahrens zur Herstellung eines Aufsatzes einer Abdeckvorrichtung für eine Knochendefektstelle die Aufnahme des ersten Datensatzes und/oder die Aufnahme des zweiten Datensatzes mit mindestens einem Verfahren, welches eine dreidimensionale Darstellung eines Knochens ermöglicht. Insbesondere erfolgen die Aufnahme des ersten Datensatzes und/oder die Aufnahme des zweiten Datensatzes mittels Tomografie, Computertomografie, digitaler Volumentomografie, Sonografie odgl..

Nach einer zusätzlichen vorteilhaften Ausgestaltung des erfindungsgemäßen Verfahrens zur Herstellung eines Aufsatzes einer Abdeckvorrichtung für eine Knochendefektstelle erfolgt die Aufnahme des Datensatzes des gesunden Knochens, nachdem der gesunde Knochen ausgewachsen ist. Dadurch ist es möglich, dass ggfls. der Idealzustand (Soll-Zustand) des Knochens dokumentiert wird, so dass bekannt ist, wie ein event. später zu regenerierender Knochen auszusehen hat. Bei Menschen sollte die Aufnahme des Datensatzes des gesunden Knochens bevorzugt im Alter von 18 bis 25 Jahren erfolgen. Selbstverständlich ist es auch denkbar, dass das im ausgewachsenen Zustand der Knochen mehrere gesunde Knochen oder das gesamte Skelett des Menschen oder des Tieres aufgezeichnet, dokumentiert und/oder gespeichert wird. Denkbar wäre auch, dass bereits zum Zeitpunkt der Aufnahme des gesunden Knochens ein Aufsatz zumindest teilweise angefertigt wird.

Nach einer diesbezüglichen vorteilhaften Ausgestaltung des erfindungsgemäßen Verfahrens zur Herstellung eines Aufsatzes einer Abdeckvorrichtung für eine Knochendefektstelle wird der Datensatz des gesunden Knochens für seine spätere Verwendung auf einem Speichermedium gespeichert (konserviert).

Nach einer zusätzlichen vorteilhaften Ausgestaltung des erfindungsgemäßen Verfahrens zur Herstellung eines Aufsatzes einer Abdeckvorrichtung für eine Knochendefektstelle wird im Fertigungsverfahren der Aufsatz mittels Fräsung gebildet.

Nach einer zusätzlichen vorteilhaften Ausgestaltung des erfindungsgemäßen Verfahrens zur Herstellung eines Aufsatzes einer Abdeckvorrichtung für eine Knochendefektstelle wird während und/oder nach der Fertigung des Aufsatzes mindestens eine Befestigungsvorrichtung für mindestens ein zu setzendes Implantat an dem Aufsatz angeordnet. Die Befestigungsvorrichtung kann beispielsweise als Aussparung ausgestaltet sein.

Nach einer diesbezüglichen vorteilhaften Ausgestaltung des erfindungsgemäßen Verfahrens zur Herstellung eines Aufsatzes einer Abdeckvorrichtung für eine Knochendefektstelle wird mindestens eine Befestigungsvorrichtung (z.B. Aussparung) durch Entfernen eines Teils des Aufsatzes, der vor dem Entfernen mittels mindestens einer Sollbruchstelle mit dem restlichen Teil des Aufsatzes verbunden ist, freigelegt. Der Zeitpunkt der Freilegung der Befestigungsvorrichtung kann dabei vor oder nach der Anordnung der Abdeckvorrichtung an der Knochendefektstelle liegen.

Nach einer zusätzlichen vorteilhaften Ausgestaltung des erfindungsgemäßen Verfahrens zur Herstellung eines Aufsatzes einer Abdeckvorrichtung für eine Knochendefektstelle wird während der Fertigung des Aufsatzes an dem Aufsatz mindestens ein Positioniermittel angeordnet, das zur Positionierung des Aufsatzes an einem an die Knochendefektstelle angrenzenden gesunden Knochen an dem Aufsatz dient und das eine dem gesunden Knochen abgewandte Wandung (Wandung im Sinne von Oberfläche) und eine dem gesunden Knochen und mit diesem zumindest teilweise korrespondierende zugewandte Wandung (Wandung im Sinne von Oberfläche) aufweist.

Nach einer diesbezüglichen vorteilhaften Ausgestaltung des erfindungsgemäßen Verfahrens zur Herstellung eines Aufsatzes einer Abdeckvorrichtung für eine Knochendefektstelle wird zwischen dem Aufsatz und einem Positioniermittel mindestens eine Sollbruchstelle angeordnet.

Nach einer zusätzlichen vorteilhaften Ausgestaltung des erfindungsgemäßen Verfahrens zur Herstellung eines Aufsatzes einer Abdeckvorrichtung für eine Knochendefektstelle wird nach der Fertigung des Aufsatzes ein Reinigungs- und/oder Sterilisationsprozess durchgeführt.

Nach einer vorteilhaften Ausgestaltung der erfindungsgemäßen Vorrichtung zur Abdeckung und/oder Rekonstruktion einer Knochendefektstelle, die einen Aufsatz aufweist, der eine dem Knochendefekt abgewandte Wandung (Wandung im Sinne von Oberfläche) und eine dem Knochendefekt zugewandte Wandung (Wandung im Sinne von Oberfläche) und ggfls. mindestens ein Fixiermittel zur Fixierung des Aufsatzes an einem Knochen aufweist, wobei der Aufsatz aus einem formstabilen Material besteht, das zumindest teilweise (am Rand) mit dem Knochen in Berührung steht, und eine dem Knochendefekt zugewandte Wandung des Aufsatzes oder eine dem Knochendefekt abgewandte Wandung des Aufsatzes der Form des regenerierten Knochens entspricht und der Aufsatz mindestens eine auftrennbare Sollbruchstelle zur Entfernung des Aufsatzes aufweist und der Aufsatz entlang einer Öffnung, die sich durch Auftrennen der mindestens einen Sollbruchstelle bildet, so dimensioniert ist, dass der Aufsatz bündig an den benachbarten gesunden Knochen einer zuvor ermittelten Knochendefektstelle abschließen kann, bestehen der Aufsatz und/oder das Fixiermittel mindestens teilweise aus einem biokompatiblen Werkstoff. Der biokompatible Werkstoff kann biotolerant, bioinert und/oder bioaktiv sein. Durch eine Sollbruchstelle wird bewirkt, dass der Aufsatz in mindestens zwei Teile zerteilbar ist, so dass er, falls eine Entfernung des Aufsatzes nach einer Knochenregeneration erwünscht ist, leicht entfernbar ist.

Nach einer zusätzlichen vorteilhaften Ausgestaltung der erfindungsgemäßen Vorrichtung ist der Werkstoff organischer Herkunft. Dies kann auch ein autogener, syngener, allogener, xenogener, synthetischer oder alloplastischer Werkstoff sein.

Nach einer zusätzlichen vorteilhaften Ausgestaltung der erfindungsgemäßen Vorrichtung bestehen der Aufsatz und/oder das Fixiermittel mindestens teilweise aus einem biodegradierbaren Werkstoff.

Nach einer zusätzlichen vorteilhaften Ausgestaltung der erfindungsgemäßen Vorrichtung können der Aufsatz und/oder das Fixiermittel mindestens teilweise aus einem resorbierbaren Werkstoff bestehen. Vorteilhafterweise kann die Resorptionszeit der starren Schale durch deren Resorptionsgradienten gesteuert werden und/oder kann die Resorptionszeit auch weniger als sechs Monate betragen, so dass zeitnah das Implantat eingesetzt werden kann. Bevorzugt werden resorbierbare Metalle oder Legierungen, insbesondere Magnesium oder Magnesiumlegierungen, eingesetzt. Die 3D-Modelle (z.B. der Aufsatz und/oder das Fixiermittel) werden bevorzugt im Laserschmelz-Verfahren (Laser-melting-Verfahren) unter Vakuum aufgebaut, wobei bevorzugt ein 3D-Drucker zum Einsatz kommt.

Nach einer zusätzlichen vorteilhaften Ausgestaltung der erfindungsgemäßen Vorrichtung bestehen der Aufsatz und/oder das Fixiermittel mindestens teilweise aus einem Polymer oder einer polymeren Verbindung.

Nach einer zusätzlichen vorteilhaften Ausgestaltung der erfindungsgemäßen Vorrichtung bestehen der Aufsatz und/oder das Fixiermittel mindestens teilweise aus Polylactid. Polylactide sind aus vielen chemisch aneinander gebundenen Milchsäuremoleküle aufgebaut und gehören zu den Polymeren. Der Vorteil von Polylactid-Kunststoffen, die auch Polymilchsäuren (PLA) genannt werden, ist, dass sie durch Wärmezufuhr verformbare Kunststoffe und biokompatibel sind.

Nach einer zusätzlichen vorteilhaften Ausgestaltung der erfindungsgemäßen Vorrichtung weist der Aufsatz eine variierende Wandstärke auf.

Nach einer diesbezüglichen vorteilhaften Ausgestaltung der erfindungsgemäßen Vorrichtung sollte die Wandstärke mindestens 0,2 mm betragen, bevorzugt 0,5 mm, jedoch zumindest so viel, so dass sich eine Formstabilität der Formschale ergibt.

Nach einer zusätzlichen vorteilhaften Ausgestaltung der erfindungsgemäßen Vorrichtung ist das Fixiermittel ein Pin, eine Schraube, ein Nagel und/oder ein Knochenkleber. Um den gesunden Knochen zu schonen, wird das Fixiermittel bevorzugt im Bereich der Knochendefektstelle angeordnet.

Nach einer zusätzlichen vorteilhaften Ausgestaltung der erfindungsgemäßen Vorrichtung weist der Aufsatz mindestens eine Fräsung (Bohrung für das Fixiermittel) auf.

Nach einer diesbezüglichen vorteilhaften Ausgestaltung der erfindungsgemäßen Vorrichtung korrespondiert die Fräsung mit dem Fixiermittel.

Nach einer zusätzlichen vorteilhaften Ausgestaltung der erfindungsgemäßen Vorrichtung weist die dem Knochendefekt zugewandte Wandung eine Oberflächenkonditionierung auf.

Nach einer diesbezüglichen vorteilhaften Ausgestaltung der erfindungsgemäßen Vorrichtung kann die Oberfläche eine Mikrostrukturierung, Poren, Osteoblastenlockstoffe, Mittel zur Förderung des Knochenwachstums und/oder BMP-haltiges Knochenersatzmittel aufweisen.

Nach einer zusätzlichen vorteilhaften Ausgestaltung der erfindungsgemäßen Vorrichtung weist der Aufsatz mindestens eine Öffnung aufweist. Dies bedeutet, dass der Aufsatz keine geschlossene Wandung aufweisen muss. Durch eine Vielzahl von Öffnungen kann der Aufsatz zumindest stellenweise eine Netzstruktur aufweisen, wobei die dem Knochendefekt abgewandte Wandung der Netzstruktur oder die dem Knochendefekt zugewandte Wandung der Netzstruktur der Form des regenerierten Knochens entspricht.

Nach einer zusätzlichen vorteilhaften Ausgestaltung der erfindungsgemäßen Vorrichtung weist der Aufsatz mindestens eine Befestigungsvorrichtung (z.B. eine Aussparung) für mindestens ein zu setzendes Implantat auf.

Nach einer diesbezüglichen vorteilhaften Ausgestaltung der erfindungsgemäßen Vorrichtung ist mindestens eine Befestigungsvorrichtung (z.B. eine Aussparung) durch einen Teil des Aufsatzes, der mittels mindestens einer Sollbruchstelle mit dem restlichen Teil des Aufsatzes verbunden ist, zumindest teilweise abgedeckt.

Nach einer zusätzlichen vorteilhaften Ausgestaltung der erfindungsgemäßen Vorrichtung ist zur Positionierung des Aufsatzes an einem an die Knochendefektstelle angrenzenden gesunden Knochen an dem Aufsatz mindestens ein Positioniermittel angeordnet ist, das eine dem gesunden Knochen abgewandte Wandung und eine dem gesunden Knochen und mit diesem zumindest teilweise korrespondierende zugewandte Wandung aufweist.

Nach einer diesbezüglichen vorteilhaften Ausgestaltung der erfindungsgemäßen Vorrichtung ist zwischen dem Aufsatz und einem Positioniermittel mindestens eine Sollbruchstelle angeordnet. Dadurch kann ein Positioniermittel, das auf dem gesunden Knochen aufliegt und somit ggfls. störend aufträgt, z.B. nach Fixierung des Aufsatzes oder nach der Regeneration des Knochens an der Knochendefektstelle, von dem eventuell verbleibenden Aufsatz entfernt werden.

Durch das erfindungsgemäße Verfahren kann eine erfindungsgemäße Vorrichtung zur Abdeckung und/oder Rekonstruktion einer Knochendefektstelle geschaffen werden, dessen Aufsatz und/oder Fixiermittel beispielsweise aus einem Werkstoff organischer und/oder anorganischer Herkunft stammt. Dies kann auch ein synthetischer Werkstoff und/oder ein Werkstoff autogener, synergener, allogener und/oder xenogener, alloplastischer, menschlicher und/oder tierischer Herkunft sein. Dabei kann auch die menschliche, tierische oder synthetische Matrix eine Form aufweisen, durch die der zwischen dem Knochen und der gewünschten Form des regenerierten Knochens befindlicher Bereich vollständig oder nahezu vollständig ausgefüllt wird. Hierzu wird dem Spender (Eigen- oder Fremdspender) z.B. ein Knochenblock entnommen, der anschließend gegebenenfalls mittels CAD/CAM modelliert wird.

Weitere Vorteile und vorteilhafte Ausgestaltungen der Erfindung sind der nachfolgenden Beschreibung, der Zeichnung und den Ansprüchen entnehmbar.

### Zeichnung

Ausführungsbeispiele des Gegenstandes der Erfindung sind in der Zeichnung dargestellt und werden im Folgenden näher erläutert. Es zeigen:
- Fig. 1: eine Darstellung einer erfindungsgemäßen Vorrichtung zur Abdeckung und/oder Rekonstruktion einer Knochendefektstelle,
- Fig. 2: eine Darstellung einer anders geformten erfindungsgemäßen Vorrichtung zur Abdeckung und/oder Rekonstruktion einer Knochendefektstelle,
- Fig. 3: eine Darstellung einer anders geformten erfindungsgemäßen Vorrichtung zur Abdeckung und/oder Rekonstruktion einer Knochendefektstelle,
- Fig. 4: eine Darstellung einer anders geformten erfindungsgemäßen Vorrichtung zur Abdeckung und/oder Rekonstruktion einer Knochendefektstelle,
- Fig. 5: einen Ausschnitt eines Aufsatzes,
- Fig. 6 bis 8: verschiedene Darstellungen eines Aufsatzes mit Positioniermitteln und
- Fig. 9: einen an einer Knochendefektstelle angeordneten Aufsatz.

### Beschreibung der Ausführungsbeispiele

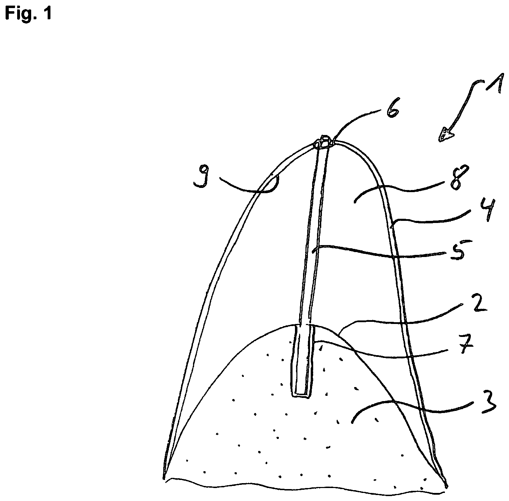

Fig. 1 zeigt eine Darstellung einer erfindungsgemäßen Vorrichtung 1 zur Abdeckung und/oder Rekonstruktion einer Knochendefektstelle 2 (Knochendefekt) eines Knochens, insbesondere eines Kieferknochens 3. Die Vorrichtung 1 besteht aus einem Aufsatz 4, der einschichtig ist, und einem Fixiermittel 5, das in Fig. 1 als Pin dargestellt in der Knochendefektstelle 2 angeordnet ist. Der Aufsatz 4 ist aus einem formstabilen Material, so dass er selbsttragend ist und keine zusätzliche Abstützung notwendig ist. Zur Fixierung des Aufsatz 4 (Formschale, starre Schale) wird das Fixiermittel 5 durch eine Bohrung 6 in den Aufsatz 4 geschoben und anschließend in die im Kieferknochen 3 angeordnete Bohrung 7 eingeführt. Die anschließende Fixierung des Aufsatzes 4 erfolgt bevorzugt über Ultraschall-Schweißen. Beim Ultraschall-Schweißen erzeugt bevorzugt ein Ultraschallgenerator eine genau definierte Frequenz, welche über eine Sonotrode gebündelt wird. Nach dem Aufsetzen des resorbierbaren Fixiermittels 5 (Pin) auf ein im Knochen vorgebohrtes Bohrloch (Bohrung 7) sorgt eine erzeugte Schwingung für eine Verflüssigung der Pinoberflächen an dessen Rändern, wodurch ein Eingleiten des Pins in das Bohrloch bewirkt wird. Durch die Änderung des Aggregatzustandes dringt der Pin auch in die knöchernen Hohlräume vor, die von einer gewöhnlichen Knochenschraube unerreichbar sind, so dass eine hohe initiale Festigkeit erzielt wird. Zudem verbindet sich der Pinkopf mit dem Aufsatz 4 und sorgt mit einem Verblockungsmechanismus für ein stabiles dreidimensionales Konstrukt. Beim Ultraschall-Schweißen wird somit das Fixiermittel 5 aufgeweicht, so dass es sich mit dem Kieferknochen 3 und dem Aufsatz 4 verbindet. Durch den fixierten Aufsatz 4 entsteht ein abgedichteter Innenraum 8 zwischen dem Kieferknochen 3 und dem Aufsatz 4, der durch die Regeneration des Knochens und/oder durch Einbringung eines Materials organischer und/oder anorganischer Herkunft, das auch ein autogener, syngener, allogener, xenogener, synthetischer und/oder alloplastischer Werkstoff sein kann, ausgefüllt wird, so dass der regenerierte Knochen oder das eingebrachte Material der Form der der Knochendefektstelle 2 zugewandten Wandung 9 (Wandung im Sinne von Oberfläche) des Aufsatzes 4 entspricht. Um den Regenerationsprozess des Kieferknochens 3 zu beschleunigen, kann die dem Knochendefekt zugewandte Wandung 9 des Aufsatzes 4 eine Oberflächenkonditionierung (z.B. eine Mikrostrukturierung, Poren, Osteoblastenlockstoffe, Mittel zur Förderung des Knochenwachstums und/oder BMP-haltiges Knochenersatzmittel) aufweisen.

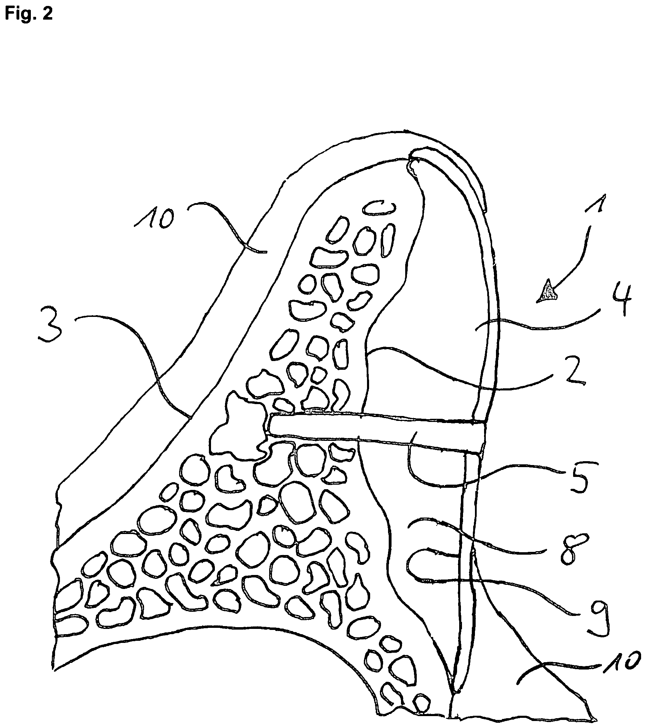

Fig. 2 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist zusätzlich das Zahnfleisch 10 angedeutet.

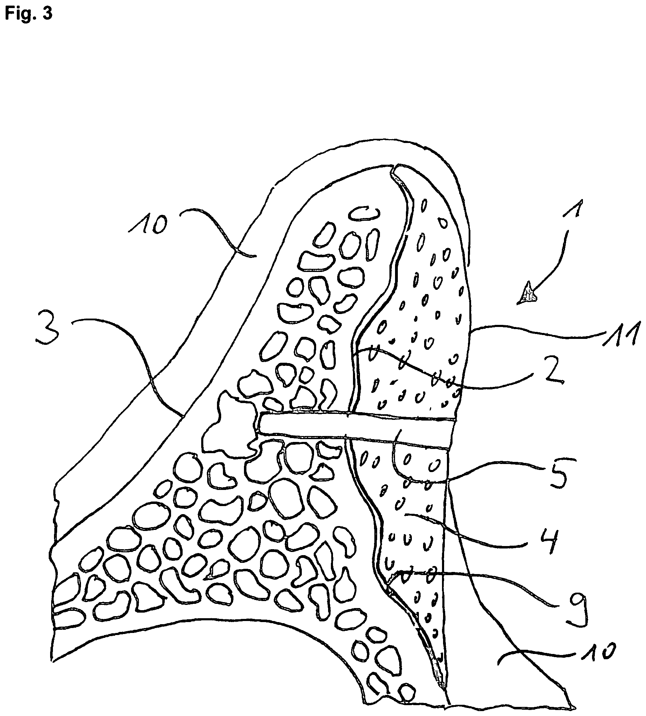

Fig. 3 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist der Aufsatz 4 als Formkörper z.B. aus menschlichen oder tierischen Knochen geformt und weist eine die dem Knochendefekt zugewandte Wandung 9 (Wandung im Sinne von Oberfläche), die an das Relief der Knochendefektstelle 2 angepasst ist, und eine dem Knochendefekt abgewandte Wandung 11 (Wandung im Sinne von Oberfläche), die der Form des regenerierten Knochens entspricht, auf.

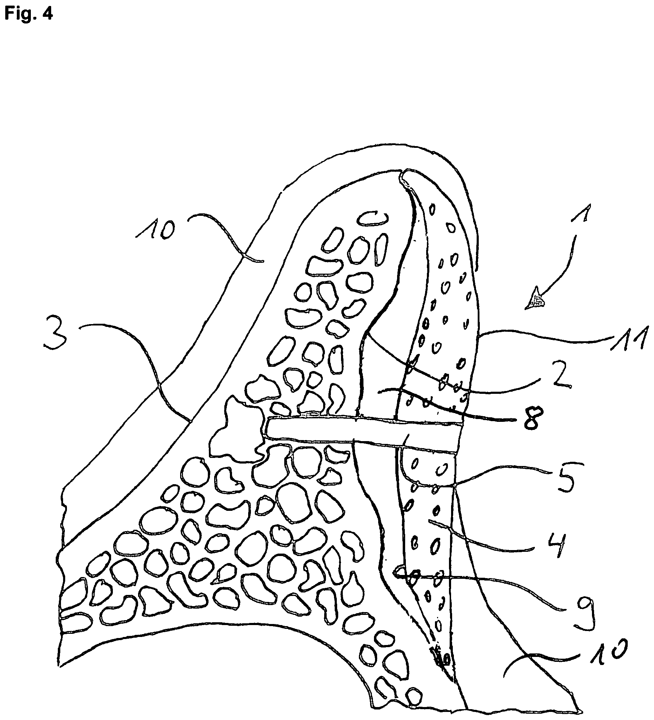

Fig. 4 zeigt eine Darstellung einer anders geformten erfindungsgemäßen Vorrichtung 1 zur Abdeckung und/oder Rekonstruktion einer Knochendefektstelle 2 (Knochendefekt) eines Knochens, insbesondere eines Kieferknochens 3. In dieser Figur ist der Aufsatz 4 als Formkörper z.B. aus menschlichen oder tierischen Knochen geformt und weist eine die dem Knochendefekt zugewandte Wandung 9 und eine dem Knochendefekt abgewandte Wandung 11, die der Form des regenerierten Knochens entspricht, auf. Zwischen der Wandung 9 und der Knochendefektstelle 2 befindet sich ein Innenraum 8, der durch die Regeneration des Knochens und/oder durch Einbringung von autogenen, syngenen, allogenen, xenogenen, synthetischen und/oder alloplastischen Material ausgefüllt wird.

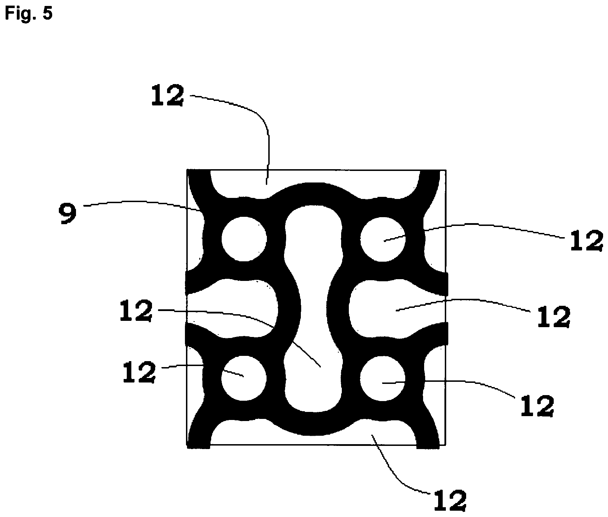

Fig. 5 zeigt einen Ausschnitt eines Aufsatzes 4, dessen dem Knochendefekt zugewandte Wandung 9 Öffnungen 12 aufweist, wodurch eine netzartige Struktur entsteht.

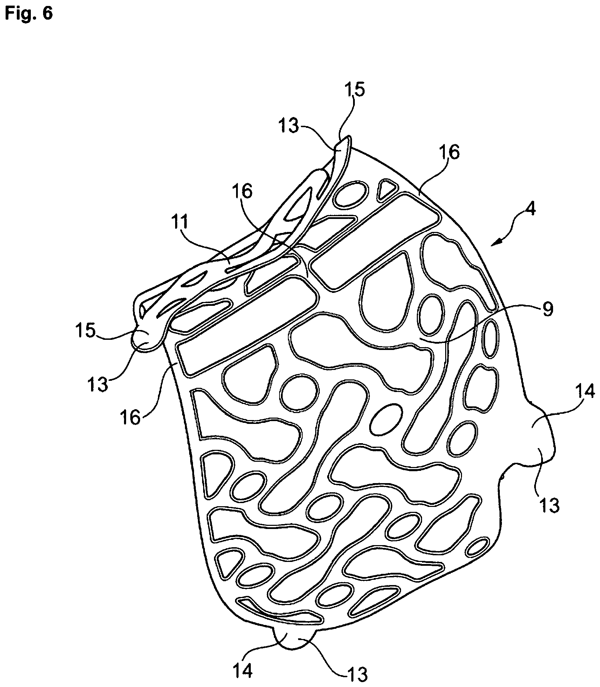

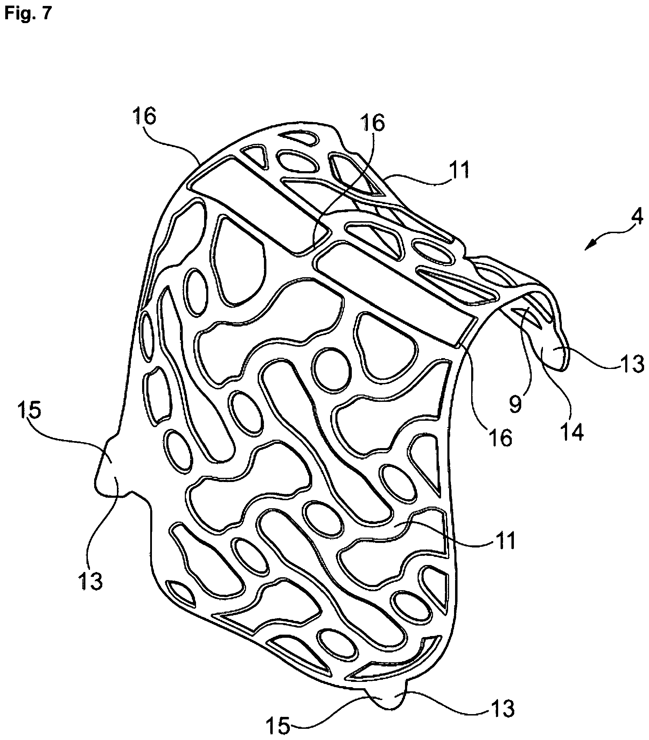

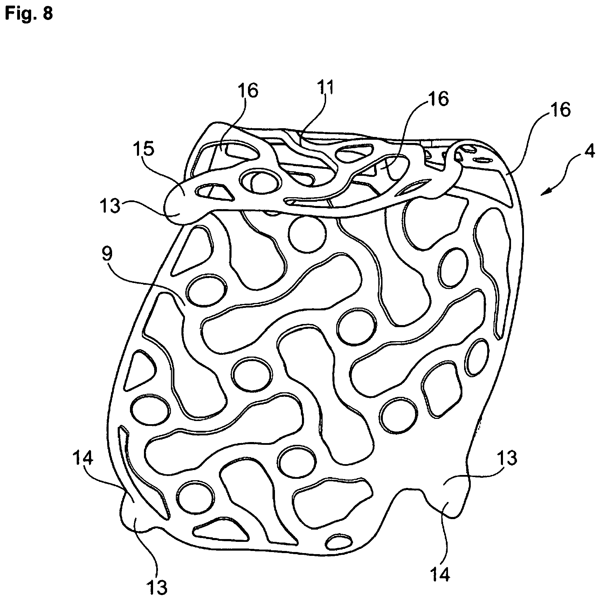

Die Fig. 6 bis 8 zeigen verschiedene Darstellungen eines Aufsatzes 4, der eine einer Knochendefektstelle zugewandte Wandung 9 und eine der Knochendefektstelle abgewandte Wandung 11 aufweist, mit Positioniermitteln 13, die eine einem gesunden Knochen zugewandte Wandung 14 und eine dem gesunden Knochen abgewandte Wandung 15 aufweisen. Bei der ordnungsgemäßen Anordnung des Aufsatzes 4 an der Knochendefektstelle berühren die einem gesunden Knochen zugewandten Wandungen 14 den gesunden Knochen, wodurch durch die Positioniermittel 13 ein einwandfreier Sitz des Aufsatzes 4 gewährleistet wird. Um den Aufsatz 4 nach der Knochenregeneration leicht entfernen zu können, weist dieser Sollbruchstellen 16 auf, wodurch er nach deren Durchtrennung für seine Entnahme in zwei Teile geteilt werden kann.

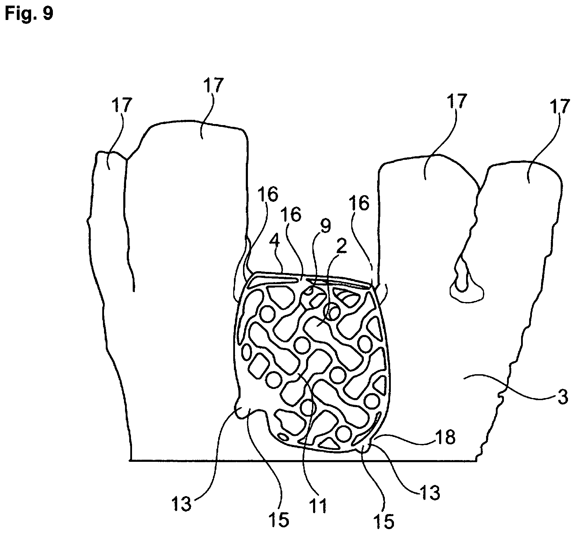

Fig. 9 zeigt einen an einer Knochendefektstelle 2 eines ausschnittsweise dargestellten Kieferknochens 3, der Zähne 17 aufweist, angeordneten Aufsatz 4. Hierdurch wird ersichtlich, dass der Aufsatz 4 bevorzugt nur im Bereich der Knochendefektstelle 2 des Kieferknochens 3 angeordnet ist, so dass er einen gesunden Knochen 18 weder überspannt noch berührt. Somit haben nur die an dem Aufsatz 4 angeordneten Positioniermittel 13 Kontakt mit dem gesunden Knochen 18.

In den Fig. 6 bis 9 ist ein Aufsatz 4 dargestellt, dessen dem Knochendefekt zugewandte Wandung 9 der Form des regenerierten Knochens entspricht. Denkbar ist auch, dass die Positioniermittel 13 derart an dem Aufsatz 4 angeordnet werden, dass dessen dem Knochendefekt abgewandte Wandung 11 der Form des regenerierten Knochens entspricht. Bewerkstelligt werden könnte dies z.B. durch Anordnung der Positioniermittel 13 an der dem Knochendefekt abgewandte Wandung 11 des Aufsatzes 4.

Alle hier dargestellten Merkmale können sowohl einzeln als auch in beliebiger Kombination miteinander erfindungswesentlich sein.

### Bezugszahlenliste

- 1: Vorrichtung
- 2: Knochendefektstelle
- 3: Kieferknochen
- 4: Aufsatz
- 5: Fixiermittel
- 6: Bohrung
- 7: Bohrung
- 8: Innenraum
- 9: Wandung
- 10: Zahnfleisch
- 11: Wandung
- 12: Öffnung
- 13: Positioniermittel
- 14: Wandung
- 15: Wandung
- 16: Sollbruchstelle
- 17: Zahn
- 18: Gesunder Knochen

## Patentansprüche

1. Verfahren zur Herstellung eines Aufsatzes (4) einer Abdeckvorrichtung für eine Knochendefektstelle (2), bestehend aus folgenden Verfahrensschritten:
- Aufnahme eines ersten Datensatzes, der die betroffene Knochendefektstelle (2) im Ist-Zustand repräsentiert,
- Vergleich des ersten Datensatzes mit einem zweiten Datensatz, der den Soll-Zustand eines an der Knochendefektstelle (2) regenerierten Knochens repräsentiert, wobei der zweite Datensatz durch Berechnung entsteht oder zu einer Zeit aufgenommen wurde, zu der der Knochen an der jetzt zu regenerierenden Stelle noch ein gesunder Knochen (18) war,
- Verwendung des ersten Datensatzes und des zweiten Datensatzes zur Planung des Aufsatzes (4), der eine dem Knochendefekt abgewandte Wandung (11) und eine dem Knochendefekt zugewandte Wandung (9) aufweist, und ggfls. mit mindestens einem Fixiermittel (5) an einem Knochen fixierbar ist,
- Umsetzung der Planung des Aufsatzes (4) in einen Planungsdatensatz und
- Zuführung des Planungsdatensatzes an ein Fertigungsverfahren, insbesondere an ein computergesteuertes Fertigungsverfahren, in dem der Aufsatz (4) aus einem formstabilen Material gebildet wird und dessen dem Knochendefekt zugewandte Wandung (9) oder dessen dem Knochendefekt abgewandte Wandung (11) der Form des regenerierten Knochens im zuvor ermittelten Soll-Zustand entspricht, wobei während der Fertigung des Aufsatzes (4) mindestens eine auftrennbare Sollbruchstelle (16) zur Entfernung des Aufsatzes (4) an dem Aufsatz (4) angeordnet wird und der Aufsatz (4) entlang einer Öffnung, die sich durch Auftrennen der mindestens einen Sollbruchstelle (16) bildet, so dimensioniert ist, dass der Aufsatz (4) bündig an den benachbarten gesunden Knochen (17) einer zuvor ermittelten Knochendefektstelle (2) abschließen kann.

2. Verfahren, nach Anspruch 1, **dadurch gekennzeichnet, dass** die Aufnahme des ersten Datensatzes die betroffene Knochendefektstelle (2) in ihrer Dreidimensionalität repräsentiert und/oder die Aufnahme des zweiten Datensatzes die Form des noch gesunden Knochens (18) in seiner Dreidimensionalität repräsentiert.

3. Verfahren, nach Anspruch 1 oder Anspruch 2, **dadurch gekennzeichnet, dass** die Aufnahme des ersten Datensatzes und/oder die Aufnahme des zweiten Datensatzes durch mindestens ein bildgebendes Verfahren erfolgt.

4. Verfahren, nach einem der vorhergehenden Ansprüche, **dadurch gekennzeichnet, dass** die Aufnahme des ersten Datensatzes und/oder die Aufnahme des zweiten Datensatzes mit mindestens einem Verfahren, welches eine dreidimensionale Darstellung eines Knochens ermöglicht, erfolgen.

5. Verfahren zur Herstellung eines Aufsatzes (4), nach einem der vorhergehenden Ansprüche, **dadurch gekennzeichnet, dass** die Aufnahme des Datensatzes des gesunden Knochens (18) erfolgt, nachdem der gesunde Knochen (18) ausgewachsen ist.

6. Verfahren zur Herstellung eines Aufsatzes (4), nach Anspruch 5, **dadurch gekennzeichnet, dass** der Datensatz des gesunden Knochens (18) für seine spätere Verwendung auf einem Speichermedium gespeichert wird.

7. Verfahren zur Herstellung eines Aufsatzes (4), nach einem der vorhergehenden Ansprüche, **dadurch gekennzeichnet, dass** im Fertigungsverfahren der Aufsatz (4) mittels Fräsung gebildet wird.

8. Verfahren zur Herstellung eines Aufsatzes (4), nach einem der vorhergehenden Ansprüche, **dadurch gekennzeichnet, dass** während der Fertigung des Aufsatzes (4) mindestens eine Befestigungsvorrichtung für mindestens ein zu setzendes Implantat an dem Aufsatz (4) angeordnet wird.

9. Verfahren zur Herstellung eines Aufsatzes (4), nach Anspruch 8, **dadurch gekennzeichnet, dass** mindestens eine Befestigungsvorrichtung durch Entfernen eines Teils des Aufsatzes (4), der vor dem Entfernen mittels mindestens einer Sollbruchstelle mit dem restlichen Teil des Aufsatzes (4) verbunden ist, freigelegt wird.

10. Verfahren zur Herstellung eines Aufsatzes (4), nach einem der vorhergehenden Ansprüche, **dadurch gekennzeichnet, dass** während der Fertigung des Aufsatzes (4) an dem Aufsatz (4) mindestens ein Positioniermittel (13) angeordnet wird, das zur Positionierung des Aufsatzes (4) an einem an die Knochendefektstelle (2) angrenzenden gesunden Knochen (18) an dem Aufsatz (4) dient und das eine dem gesunden Knochen (18) abgewandte Wandung (15) und eine dem gesunden Knochen (18) und mit diesem zumindest teilweise korrespondierende zugewandte Wandung (14) aufweist.

11. Verfahren zur Herstellung eines Aufsatzes (4), nach Anspruch 10, **dadurch gekennzeichnet, dass** zwischen dem Aufsatz (4) und einem Positioniermittel (13) mindestens eine Sollbruchstelle (16) angeordnet wird.

12. Verfahren zur Herstellung eines Aufsatzes (4), nach einem der vorhergehenden Ansprüche, **dadurch gekennzeichnet, dass** nach der Fertigung des Aufsatzes (4) ein Reinigungs- und/oder Sterilisationsprozess durchgeführt wird.

13. Vorrichtung (1) zur Abdeckung und/oder Rekonstruktion einer Knochendefektstelle (2),
- mit einem Aufsatz (4), der eine dem Knochendefekt abgewandte Wandung (11) und eine dem Knochendefekt zugewandte Wandung (9) aufweist,
- mit ggfls. mindestens einem Fixiermittel (5) zur Fixierung des Aufsatzes (4) an einem Knochen,
wobei der Aufsatz (4) aus einem formstabilen Material besteht, das zumindest teilweise dazu vorgesehen ist mit dem Knochen in Berührung zu stehen steht, und eine dem Knochendefekt zugewandte Wandung (9) des Aufsatzes (4) oder eine dem Knochendefekt abgewandte Wandung (11) des Aufsatzes (4) der Form des regenerierten Knochens entspricht,
**dadurch gekennzeichnet,**
**dass** der Aufsatz (4) mindestens eine auftrennbare Sollbruchstelle (16) zur Entfernung des Aufsatzes (4) aufweist und der Aufsatz (4) entlang einer Öffnung, die sich durch Auftrennen der mindestens einen Sollbruchstelle (16) bildet, so dimensioniert ist, dass der Aufsatz (4) bündig an den benachbarten gesunden Knochen (17) einer zuvor ermittelten Knochendefektstelle (2) abschließen kann.

14. Vorrichtung (1), nach Anspruch 13, **dadurch gekennzeichnet, dass** der Aufsatz (4) und/oder das Fixiermittel (5) mindestens teilweise aus einem biokompatiblen Werkstoff bestehen.

15. Vorrichtung (1), nach Anspruch 13 oder Anspruch 14, **dadurch gekennzeichnet, dass** der Werkstoff des Aufsatzes (4) organischer Herkunft ist und der Werkstoff eines Fixiermittels (5) organischer oder anorganischer Herkunft ist.

16. Vorrichtung (1), nach einem der Ansprüche 13 bis 15, **dadurch gekennzeichnet, dass** der Aufsatz (4) und/oder das Fixiermittel (5) mindestens teilweise aus einem biodegradierbaren Werkstoff bestehen.

17. Vorrichtung (1), nach einem der Ansprüche 13 bis 16, **dadurch gekennzeichnet, dass** der Aufsatz (4) und/oder das Fixiermittel (5) mindestens teilweise aus einem resorbierbaren Werkstoff bestehen.

18. Vorrichtung (1), nach einem der Ansprüche 13 bis 17, **dadurch gekennzeichnet, dass** der Aufsatz (4) und/oder das Fixiermittel (5) mindestens teilweise aus einem Polymer oder einer polymeren Verbindung bestehen.

19. Vorrichtung (1), nach einem der Ansprüche 13 bis 18, **dadurch gekennzeichnet, dass** der Aufsatz (4) und/oder das Fixiermittel (5) mindestens teilweise aus Polylactid bestehen.

20. Vorrichtung (1), nach einem der Ansprüche 13 bis 19, **dadurch gekennzeichnet, dass** der Aufsatz (4) eine variierende Wandstärke aufweist.

21. Vorrichtung (1), nach Anspruch 20, **dadurch gekennzeichnet, dass** die Wandstärke mindestens 0,2 mm beträgt.

22. Vorrichtung (1), nach einem der Ansprüche 13 bis 22, **dadurch gekennzeichnet, dass** das Fixiermittel (5) ein Pin, eine Schraube, ein Nagel und/oder ein Knochenkleber ist.

23. Vorrichtung (1), nach einem der Ansprüche 13 bis 22, **dadurch gekennzeichnet, dass** der Aufsatz (4) mindestens eine Fräsung aufweist.

24. Vorrichtung (1), nach Anspruch 23, **dadurch gekennzeichnet, dass** die Fräsung mit dem Fixiermittel (5) korrespondiert.

25. Vorrichtung (1), nach einem der Ansprüche 13 bis 25, **dadurch gekennzeichnet, dass** die dem Knochendefekt zugewandte Wandung (9) eine Oberflächenkonditionierung aufweist.

26. Vorrichtung (1), nach Anspruch 25, **dadurch gekennzeichnet, dass** die Oberflächenkonditionierung eine Mikrostrukturierung, Poren, Osteoblastenlockstoffe, Mittel zur Förderung des Knochenwachstums und/oder BMP-haltiges Knochenersatzmittel aufweist.

27. Vorrichtung (1), nach einem der Ansprüche 13 bis 26, **dadurch gekennzeichnet, dass** der Aufsatz (4) mindestens eine Öffnung aufweist.

28. Vorrichtung (1), nach einem der Ansprüche 13 bis 27, **dadurch gekennzeichnet, dass** der Aufsatz (4) mindestens eine Befestigungsvorrichtung für mindestens ein zu setzendes Implantat aufweist.

29. Vorrichtung (1), nach Anspruch 28, **dadurch gekennzeichnet, dass** mindestens eine Befestigungsvorrichtung durch einen Teil des Aufsatzes (4), der mittels mindestens einer Sollbruchstelle mit dem restlichen Teil des Aufsatzes (4) verbunden ist, zumindest teilweise abgedeckt ist.

30. Vorrichtung (1), nach einem der Ansprüche 13 bis 29, **dadurch gekennzeichnet, dass** zur Positionierung des Aufsatzes (4) an einem an die Knochendefektstelle (2) angrenzenden gesunden Knochen (18) an dem Aufsatz (4) mindestens ein Positioniermittel (13) angeordnet ist, das eine dem gesunden Knochen abgewandte Wandung (15) und eine dem gesunden Knochen (18) und mit diesem zumindest teilweise korrespondierende zugewandte Wandung (14) aufweist.

31. Vorrichtung (1), nach Anspruch 30, **dadurch gekennzeichnet, dass** zwischen dem Aufsatz (4) und einem Positioniermittel (13) mindestens eine Sollbruchstelle (16) angeordnet ist.

## Claims

1. A method for producing a top attachment (4) of a covering device for a bone defect site (2), the method consisting of the following process steps:
- recording a first data set which represents the involved bone defect site (2) in its current state,
- comparing said first data set with a second data set which represents the target state of regenerated bone at the bone defect site (2), the second data set being either obtained by calculation or having been recorded at a time when the bone at the site which is currently to be regenerated had still been healthy bone (18),
- utilising said first data set and said second data set for the purpose of planning the top attachment (4), which has a wall (11) facing away from the bone defect, as well as a wall (9) facing towards the bone defect, and which is suitable, if and when necessary, for being fixed onto a bone by at least one fixing means (5),
- implementing the planning of the top attachment (4) in the form of a planning data set, and
- supplying said planning data set to a manufacturing process, in particular to a computer-controlled manufacturing process, in which the top attachment (4) is made of a dimensionally stable material and its wall (9) facing towards the bone defect or its wall (11) facing away from the bone defect corresponds to the shape of the regenerated bone in its previously determined target state, wherein concurrently with the manufacture of the top attachment (4) at least one predetermined breaking point (16) is disposed on said top attachment (4) which is designed for removing said top attachment (4) and wherein the top attachment (4) is dimensioned in such a way along an opening that forms upon breaking of the predetermined breaking point (16), that the top attachment (4) may be flush with the healthy bone (17) adjoining a previously determined bone defect site (2).

2. The method as claimed in claim 1, **characterised in that** the recording of the first data set represents the involved bone defect site (2) in its three-dimensionality and/or the recording of the second data set represents the shape of the still healthy bone (18) in its three-dimensionality.

3. The method as claimed in claim 1 or claim 2, **characterised in that** the recording of the first data set and/or the recording of the second data set is/are obtained by at least one imaging technique.

4. The method as claimed in any of the preceding claims, **characterised in that** the recording of the first data set and/or the recording of the second data set is/are obtained by employing a method permitting three-dimensional bone representation.

5. A method for producing a top attachment (4) as claimed in any of the preceding claims, **characterised in that** the recording of the data set of the healthy bone (18) is taken after the healthy bone (18) has grown to its full size.

6. A method for producing a top attachment (4) as claimed in claim 5, **characterised in that** the data set of the healthy bone (18) is stored on a storage means for later use.

7. The method for producing a top attachment (4) as claimed in any of the preceding claims, **characterised in that** in the course of the manufacturing process, the top attachment (4) is realised by means of milling.

8. The method for producing a top attachment (4) as claimed in any of the preceding claims, **characterised in that** during the manufacture of the top attachment (4) at least one fastening device for at least one implant to be inserted is arranged on said top attachment (4).

9. The method for producing a top attachment (4) as claimed in claim 8, **characterised in that** at least one fastening device is exposed by removing a part of the top attachment (4) which prior to its removal is connected to the remaining part of the top attachment (4) by means of at least one predetermined breaking point.

10. The method for producing a top attachment (4) as claimed in any of the preceding claims, **characterised in that** during the manufacture of the top attachment (4) at least one positioning means (13) is arranged on said top attachment (4) which serves for the purpose of positioning the top attachment (4) on a healthy bone (18) adjoining the bone defect site (2) and which has a wall (15) facing away from the healthy bone (18) and a wall (14) facing towards, and corresponding at least partially with, the healthy bone (18).

11. The method for producing a top attachment (4) as claimed in claim 10, **characterised in that** the top attachment (4) and a positioning means (13) have at least one predetermined breaking point (16) arranged therebetween.

12. The method for producing a top attachment (4) as claimed in any of the preceding claims, **characterised in that** a cleaning process and/or a sterilisation process is/are carried out after the manufacture of the top attachment (4).

13. A device 1 for covering and/or reconstructing a bone defect site 2,
- including a top attachment (4), which has a wall (11) facing away from the bone defect and a wall (9) facing towards the bone defect,
- including, if and when necessary, at least one fixing means (5) for fixing the top attachment (4) onto a bone,
wherein the top attachment (4) is made of a dimensionally stable material which is at least partially designed to be in contact with the bone and wherein a wall (9) of the top attachment (4) facing towards the bone defect or a wall (11) of the top attachment (4) facing away from the bone defect corresponds to the shape of the regenerated bone, **characterised in that**
the top attachment (4) has at least one predetermined breaking point (16) disposed thereon which is designed for removing said top attachment (4) and wherein the top attachment (4) is dimensioned in such a way along an opening that forms upon breaking of the predetermined breaking point (16),
that the top attachment (4) may be flush with the healthy bone (17) adjoining a previously determined bone defect site (2).

14. The device (1) as claimed in claim 13, **characterised in that** the top attachment (4) and/or the fixing means (5) consist(s) at least partially of a biocompatible material.

15. The device (1) as claimed in claim 13 or claim 14, **characterised in that** the material of the top attachment (4) is of organic origin and/or the material of the fixing means (5) is of organic or inorganic origin.

16. The device (1) as claimed in any one of claims 13 to 15, **characterised in that** the top attachment (4) and/or the fixing means (5) consist(s) at least partially of a biodegradable material.

17. The device (1) as claimed in any one of claims 13 to 16, **characterised in that** the top attachment (4) and/or the fixing means (5) consist(s) at least partially of a resorbable material.

18. The device (1) as claimed in any one of claims 13 to 17, **characterised in that** the top attachment (4) and/or the fixing means (5) consist(s) at least partially of a polymer or a polymeric compound.

19. The device (1) as claimed in any one of claims 13 to 18, **characterised in that** the top attachment (4) and/or the fixing means (5) consist(s) at least partially of polyactide.

20. The device (1) as claimed in any one of claims 13 to 19, **characterised in that** the top attachment (4) has a varying wall thickness.

21. The device (1) as claimed in claim 20, **characterised in that** the wall thickness is at least 0.2 mm.

22. The device (1) as claimed in any one of claims 13 to 22, **characterised in that** the fixing means (5) is a pin, a screw, a nail and/or a bone adhesive.

23. The device (1) as claimed in any one of claims 13 to 22, **characterised in that** the top attachment (4) has at least one milling.

24. The device (1) as claimed in claim 23, **characterised in that** the milling corresponds to the fixing means (5).

25. The device (1) as claimed in any one of claims 13 to 25, **characterised in that** the wall (9) facing towards the bone defect has a surface conditioning.

26. The device (1) as claimed in claim 25, **characterised in that** the surface conditioning has a micro-structuring, pores, osteoblast attractants, substances for encouraging bone growth, and/or a BMP-containing bone substitute.

27. The device (1) as claimed in any one of claims 13 to 26, **characterised in that** the top attachment (4) has at least one opening.

28. The device (1) as claimed in any one of claims 13 to 27, **characterised in that** the top attachment (4) has at least one fastening device for at least one implant to be inserted.

29. The device (1) as claimed in claim 28, **characterised in that** at least one fastening device is at least partially covered by a part of the top attachment (4) which is connected to the remaining part of the top attachment (4) by means of at least one predetermined breaking point.

30. The device (1) as claimed in any one of claims 13 to 29, **characterised in that** for the purpose of positioning the top attachment (4) on healthy bone (18) adjoining the bone defect site (2), said top attachment (4) has at least one positioning means (13) arranged thereon which has a wall (15) facing away from the healthy bone and a wall (14) facing towards, and corresponding at least partially with, the healthy bone (18).

31. The device (1) as claimed in claim 30, **characterised in that** the top attachment (4) and a positioning means (13) have at least one predetermined breaking point (16) arranged therebetween.

## Revendications

1. Procédé destiné à fabriquer un support (4) d'un dispositif de recouvrement pour une zone de défaut osseux (2), lequel comprend les étapes suivantes :
- la collecte d'un premier ensemble de données qui représente la zone de défaut osseux (2) concernée à l'état initial,
- la comparaison du premier ensemble de données avec un deuxième ensemble de données qui représente l'état final visé d'un os régénéré au niveau de la zone de défaut osseux (2), le deuxième ensemble de données étant obtenu par calcul ou a été collecté à un moment où l'os au niveau de la zone qui doit être maintenant régénérée était encore un os sain (18),
- l'utilisation du premier ensemble de données et du deuxième ensemble de données en vue de concevoir le support (4) qui présente une paroi (11) opposée au défaut osseux et une paroi (9) tournée vers le défaut osseux et qui, le cas échéant, peut être fixé à un os à l'aide d'au moins un moyen de fixation (5),
- la réalisation de la conception du support (4) dans un ensemble de données de conception et
- l'importation de l'ensemble de données de conception dans un procédé de fabrication, en particulier un procédé de fabrication assisté par ordinateur, dans lequel le support (4) est constitué en un matériau à forme stable et dont la paroi (9) tournée vers le défaut osseux ou dont la paroi (11) opposée au défaut osseux correspond à la forme de l'os régénéré à l'état final visé déterminé préalablement, pendant la fabrication du support (4) étant disposé sur le support (4) au moins un point destiné à la rupture (16) séparable en vue de retirer ledit support (4), et le support (4) étant, le long d'une ouverture qui se forme lors de la séparation dudit au moins un point destiné à la rupture (16), dimensionné de telle sorte que ledit support (4) peut reposer contre l'os sain (17) contigu à une zone de défaut osseux (2) déterminée préalablement en formant avec celle-ci une surface plane.

2. Procédé selon la revendication 1, **caractérisé en ce que** la collecte du premier ensemble de données représente la zone de défaut osseux (2) concernée dans sa tridimensionnalité et/ou **en ce que** la collecte du deuxième ensemble de données représente la forme de l'os encore sain (18) dans sa tridimensionnalité.

3. Procédé selon la revendication 1 ou selon la revendication 2, **caractérisé en ce que** la collecte du premier ensemble de données et/ou la collecte du deuxième ensemble de données est obtenue par au moins un procédé d'imagerie.

4. Procédé selon l'une quelconque des revendications précédentes, **caractérisé en ce que** la collecte du premier ensemble de données et/ou la collecte du deuxième ensemble de données est obtenue par au moins un procédé qui rend possible une représentation tridimensionnelle d'un os.

5. Procédé destiné à fabriquer un support (4) selon l'une quelconque des revendications précédentes, **caractérisé en ce que** la collecte de l'ensemble de données de l'os sain (18) est effectuée après la croissance de l'os sain (18).

6. Procédé destiné à fabriquer un support (4) selon la revendication 5, **caractérisé en ce que** l'ensemble de données de l'os sain (18) est stocké sur un support de stockage en vue de son utilisation ultérieure.

7. Procédé destiné à fabriquer un support (4) selon l'une quelconque des revendications précédentes, **caractérisé en ce que** dans ledit procédé de fabrication, le support (4) est réalisé par fraisage.

8. Procédé destiné à fabriquer un support (4) selon l'une quelconque des revendications précédentes, **caractérisé en ce que** pendant la fabrication du support (4), au moins un dispositif de fixation pour au moins un implant à poser est disposé sur le support (4).

9. Procédé destiné à fabriquer un support (4) selon la revendication 8, **caractérisé en ce qu'**au moins un dispositif de fixation est libéré lorsque l'on retire une partie du support (4) qui, avant d'être retirée, est reliée à la partie restante du support (4) par au moins un point destiné à la rupture.

10. Procédé destiné à fabriquer un support (4) selon l'une quelconque des revendications précédentes, **caractérisé en ce que** pendant la fabrication du support (4) est disposé, sur le support (4), au moins un moyen de positionnement (13) qui sert à positionner ledit support (4) contre un os sain (18) contigu à la zone de défaut osseux (2) et qui présente une paroi (15) opposée à l'os sain (18) ainsi qu'une paroi (14) tournée vers l'os sain (18) et correspondant au moins partiellement à celui-ci.

11. Procédé destiné à fabriquer un support (4) selon la revendication 10, **caractérisé en ce qu'**au moins un point destiné à la rupture (16) est disposé entre le support (4) et un moyen de positionnement (13).

12. Procédé destiné à fabriquer un support (4) selon l'une quelconque des revendications précédentes, **caractérisé en ce qu'**un processus de nettoyage et/ou de stérilisation est effectué après la fabrication du support (4).

13. Dispositif (1) destiné à recouvrir et/ou à reconstruire une zone de défaut osseux (2),
- pourvu d'un support (4) qui présente une paroi (11) opposée au défaut osseux et une paroi (9) tournée vers le défaut osseux,
- pourvu, le cas échéant, d'au moins un moyen de fixation (5) destiné à fixer le support (4) à un os,
le support (4) étant constitué en un matériau à forme stable qui est prévu au moins en partie pour être en contact avec l'os, et une paroi (9) du support (4) tournée vers le défaut osseux ou une paroi (11) du support (4) opposée au défaut osseux correspondant à la forme de l'os régénéré, **caractérisé en ce que**
le support (4) présente au moins un point destiné à la rupture (16) séparable en vue de retirer ledit support (4), et le support (4) est, le long d'une ouverture qui se forme lors de la séparation dudit au moins un point destiné à la rupture (16), dimensionné de telle sorte que ledit support (4) peut reposer contre l'os sain (17) contigu à une zone de défaut osseux (2) déterminée préalablement en formant avec celle-ci une surface plane.

14. Dispositif (1) selon la revendication 13, **caractérisé en ce que** le support (4) et/ou le moyen de fixation (5) sont constitués au moins en partie en un matériau biocompatible.

15. Dispositif (1) selon la revendication 13 ou selon la revendication 14, **caractérisé en ce que** le matériau du support (4) est d'origine organique et/ou que le matériau d'un moyen de fixation (5) est d'origine organique ou inorganique.

16. Dispositif (1) selon l'une quelconque des revendications 13 à 15, **caractérisé en ce que** le support (4) et/ou le moyen de fixation (5) sont constitués au moins en partie en un matériau biodégradable.

17. Dispositif (1) selon l'une quelconque des revendications 13 à 16, **caractérisé en ce que** le support (4) et/ou le moyen de fixation (5) sont constitués au moins en partie en un matériau résorbable.

18. Dispositif (1) selon l'une quelconque des revendications 13 à 17, **caractérisé en ce que** le support (4) et/ou le moyen de fixation (5) sont constitués au moins en partie en un polymère ou en un composé polymère.

19. Dispositif (1) selon l'une quelconque des revendications 13 à 18, **caractérisé en ce que** le support (4) et/ou le moyen de fixation (5) sont constitués au moins en partie en polylactide.

20. Dispositif (1) selon l'une quelconque des revendications 13 à 19, **caractérisé en ce que** le support (4) présente une épaisseur de paroi variable.

21. Dispositif (1) selon la revendication 20, **caractérisé en ce que** l'épaisseur de paroi est d'au moins 0,2 mm.

22. Dispositif (1) selon l'une quelconque des revendications 13 à 22, **caractérisé en ce que** le moyen de fixation (5) est une broche, une vis, un clou et/ou une colle à os.

23. Dispositif (1) selon l'une quelconque des revendications 13 à 22, **caractérisé en ce que** le support (4) présente au moins un fraisage.

24. Dispositif (1) selon la revendication 23, **caractérisé en ce que** le fraisage correspond au moyen de fixation (5).

25. Dispositif (1) selon l'une quelconque des revendications 13 à 25, **caractérisé en ce que** la paroi (9) tournée vers le défaut osseux présente un conditionnement de surface.

26. Dispositif (1) selon la revendication 25, **caractérisé en ce que** le conditionnement de surface présente une microstructuration, des pores, des attractants pour ostéoblastes, des moyens favorisant la croissance osseuse et/ou des substituts osseux contenant du BMP.

27. Dispositif (1) selon l'une quelconque des revendications 13 à 26, **caractérisé en ce que** le support (4) présente au moins une ouverture.

28. Dispositif (1) selon l'une quelconque des revendications 13 à 27, **caractérisé en ce que** le support (4) présente au moins un dispositif de fixation pour au moins un implant à poser.

29. Dispositif (1) selon la revendication 28, **caractérisé en ce qu'**au moins un dispositif de fixation est recouvert au moins partiellement par une partie du support (4) qui est reliée à la partie restante dudit support (4) par au moins un point destiné à la rupture.

30. Dispositif (1) selon l'une quelconque des revendications 13 à 29, **caractérisé en ce qu'**en vue de positionner le support (4) sur un os sain (18) contigu à la zone de défaut osseux (2) est disposé sur ledit support (4) au moins un moyen de positionnement (13) qui présente une paroi (15) opposée à l'os sain ainsi qu'une paroi (14) tournée vers l'os sain (18) et correspondant au moins partiellement à celui-ci.

31. Dispositif (1) selon la revendication 30, **caractérisé en ce qu'**au moins un point destiné à la rupture (16) est disposé entre le support (4) et un moyen de positionnement (13).
